## Europäisches Patentamt
## European Patent Office
## Office européen des brevets

(19)

(11) Publication number: **0 014 530**
**B1**

(12)

# EUROPEAN PATENT SPECIFICATION

(45) Date of publication of patent specification: **14.03.84**

(51) Int. Cl.³: **G 01 N 33/54, C 12 Q 1/00**

(21) Application number: **80300172.6**

(22) Date of filing: **18.01.80**

(54) **Method for detecting and determining proteinaceous specific binding agents and materials bindable thereto, test composition and testkit therefor.**

(30) Priority: **18.01.79 GB 7901866**

(43) Date of publication of application:
**20.08.80 Bulletin 80/17**

(45) Publication of the grant of the patent:
**14.03.84 Bulletin 84/11**

(84) Designated Contracting States:
**AT BE CH DE FR GB IT LU NL SE**

(56) References cited:
**DE - A - 2 411 884**
**FR - A - 2 206 859**
**FR - A - 2 400 204**
**GB - A - 1 470 955**
**US - A - 3 887 698**

**CHEMICAL ABSTRACTS, vol. 87, no. 3, July 18, 1977, Page 426, abstract 20373y. Columbus, Ohio, USA ROSENQUIST, E et al. "Immune lysis of spin label loaded liposomes incorporating cardiolipin; a new sensitive method for detecting anticardiolipin antibodies in syphilis serology".**

(73) Proprietor: **UNILEVER PLC**
**Unilever House Blackfriars P O Box 68**
**London EC4P 4BQ (GB)**
(84) **GB**
(73) Proprietor: **UNILEVER NV**
**Burgemeester s'Jacobplein 1 P.O. Box 760**
**NL-3000 DK Rotterdam (NL)**
(84) **BE CH DE FR IT LU NL SE AT**

(72) Inventor: **Davis, Paul James**
**30 Marriott's Close**
**Felmersham Bedfordshire (GB)**

(74) Representative: **Stancliffe, Terence Christopher et al,**
**UNILEVER PLC Patents Division P.O. Box 68**
**Unilever House**
**London EC4P 4BQ (GB)**

(56) References cited:
**CHEMICAL ABSTRACTS, vol. 78, no. 19, May 14, 1973 page 163, abstract 120694m. Columbus, Ohio, USA KATAOKA, T. et al. "Release of macromolecular markers (enzymes) from liposomes treated with antibody and complement. Attempt at correlation with electron microscopic observations"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Method for detecting and determining proteinaceous specific binding agents and materials bindable thereto, test composition and testkit therefor

This invention concerns improvements in or relating to processes and materials for detecting and determining proteinaceous specific binding agents and materials bindable thereto. The invention has particular application for example to the detection and determination of antibodies and their corresponding antigens or haptens, among other materials which take part in avid specific binding reactions.

Known analytical methods exist for detecting and determining antigens, haptens and antibodies of a wide variety of types, and other substances which take part in avid specific binding reactions, such as avidin (which binds biotin), thyroxin-binding globulin, and transcortin (which binds cortisol). Among the large number of antigens, haptens and antibodies for which analytical methods have been devised are for example insulin and anti-insulin; human chorionic gonadotrophin and antibodies thereto; antibodies against disease-producing organisms such as *Toxoplasma*, *Entamoeba* and *Treponema*, and antigenic components of such organisms themselves; virus components such as hepatitis antigen and antibodies thereto; normal and abnormal tissue components, derivatives thereof and antibodies thereto, such as proteins including immunoglobulins themselves such as IgG and IgM, and degradation products thereof, such as Fc fragments of IgG, low molecular weight hormones, such as oestradiol; nutritional factors such as folate; and drugs such as opium alkaloids and cardiac steroids such as digoxin.

Among the known techniques for carrying out assay of such materials are radioimmunoassays, and immunoassays dependent on the detection of something other than a radioactive label, for example a fluorescent material, an electron spin label or an enzyme.

US—A—3,654,090, 3,791,932, 3,839,153, 3,850,752 and 3,879,262 are representative of immunoassays dependent on the use of a covalent conjugate of an enzyme with one component of a pair of specific binding reaction components of interest in the assays in question. Assays of this type have proved to be in many ways useful and convenient. However, there is a need for assay materials and systems with a potential for greater sensitivity, and which can be arranged into assay methods which are flexible and convenient in use.

According to this invention there are provided materials for the detection or determination of an analyte which is a component of the reaction between proteinaceous binding agents and materials bindable thereto, in which one of the reaction components, i.e. a binding agent or a material bindable thereto, is fixed or coupled to the surface of vesicles which contain a quantity of enzyme-containing aqueous material which is occluded from contact with the bulk of the suspending medium in the absence of lytic conditions, and in which one of the reaction components is immobilised on a solid carrier in a form which is able to accept the binding of a complementary reaction component when fixed or coupled to the vesicles. This latter immobilised component is referred to herein as an immunoadsorbent. The materials for the detection or determination of an analyte which is one of the reaction components, can conveniently be in the form of a test kit having dosed quantities of the components in a form suitable for their application to assay. They can also include a lytic agent for releasing or rendering accessible the contents of vesicles, for reacting with vesicles which have taken part in a detection or determination reaction, and a substrate yielding detectable product upon which the enzyme can react once released or rendered accessible by the lytic agent.

The binding specificities of the reaction components fixed or coupled to the surface of the vesicles and immobilised on the solid carrier can take any of a very wide variety of forms and combinations which in themselves can be analogous to the specificities and configurations of known binding assays and immunoassays and in themselves do not constitute this invention. They will be determined by the identity of the material which it is desired to assay and by the desired assay configuration. A number of examples of these are given below to facilitate understanding of the present invention and illustrate particular applications of it.

The materials provided according to the invention are used according to the invention in a binding assay method for one of the above-mentioned reaction components in which both the vesicle-fixed or -coupled reaction component mentioned above, and the immunoadsorbent capable of accepting the binding of vesicles which have a complementary reaction component bound or coupled thereto, are used, and in which the detection or determination of a portion of the vesicles which has reacted in an amount which is dependent upon the amount of any analyte present is carried out by lysing any such reacted vesicles with the lytic agent so as to release or render accessible the enzyme contained therein, and determining the amount or presence of any such released enzyme by reaction with a substrate that gives rise to a detectable product.

In the materials according to the invention the specificity of the immunoadsorbent can be either complementary or homologous to that of the reaction component fixed or coupled to the vesicles, i.e. they can bind together, or they can bind to similar or common binding partners, respectively.

In one embodiment, an assay method according to the invention comprises:

(1) immobilising an antibody B to an antigen A (to be detected or determined), which can for example be an amphipathic antigen, onto an activated solid surface to form an immunoadsorbent;

(2) fixing any antigen A present in the test sample to surface membranes of vesicles, preferably liposomes, filled with an enzyme marker;

(3) bringing said vesicles carrying antigen A into intimate contact with the immunoadsorbent to bind any antigen A fixed on the vesicles, to B;

(4) releasing the enzyme marker by causing lysis of the vesicle;

(5) measuring the level of enzyme marker.

In an embodiment particularly applicable to monovalent antigens, an assay method according to the invention can comprise:

(1) immobilising antigen molecules, A, similar to those to be determined, on an activated solid surface to form an immunoadsorbent;

(2) fixing antibody B specific to antigen A, on the surface membranes of vesicles filled with a marker enzyme;

(3) bringing the vesicles carrying antibody B into intimate contact with a test sample which possibly contains antigen A, and then with the immobilised antigen A in the form of the immunoadsorbent;

(4) releasing the marker enzyme by causing lysis of any vesicles carrying B bound to immobilised A;

(5) measuring the level of marker enzyme by causing formation of products of the enzymatic reaction between the marker enzyme and an appropriate substrate.

If the sample contains analyte (antigen A), this will react with B fixed on the vesicles; then there will be less free B available to react with the immobilised A, and the level measured in step 5 will be correspondingly reduced as compared with the level found in the absence of analyte in the sample.

When the analyte antigen A to be determined is at least bivalent, a preferred process according to the invention can involve the following steps:

(1) immobilising an antibody B (to analyte antigen A), onto an activated solid surface to form an immunoadsorbent;

(2) binding any analyte antigen A present in the test sample to B by bringing the test sample into intimate contact with the immunoadsorbent;

(3) binding to analyte antigen A a bi-functional complex B′ fixed on the surface membranes of vesicles filled with an enzyme marker and carrying an amphipathic antigen;

(4) releasing the enzyme marker by causing lysis of the vesicle;

(5) measuring the level of enzyme marker, e.g. as described above.

In step 3 of the above process according to the invention, the amphipathic antigen is bound to the bifunctional complex B′ which consists of an antibody C specific to the amphipathic antigen and an antibody D to antigen A. (Antibody D can be a preparation similar to antibody B.) Antibody D can alternatively be capable of binding to antigen A through different antigenic determinants of antigen A, as compared with the specificity of antibody B.

An embodiment of the process of the invention for detecting or determining an antibody can involve the following steps:

(1) immobilising an antigen Y to which antibody X is specific onto an activated solid surface to form an immunoadsorbent;

(2) binding any antibody X present in the test sample to the immobilised antigen Y by bringing the test sample into intimate contact with the immunoadsorbent;

(3) binding X to a bifunctional complex B″ consisting of a heterologous antiglobulin Z specific to the antibody type and species to which X belongs, and an antibody C specific to an amphipathic antigen fixed on the surface membrane of vesicles filled with a marker enzyme;

(4) releasing the marker enzyme by causing lysis of the vesicle, and

(5) measuring the level of marker, e.g. as described above.

It will be understood that in this embodiment according to the invention the sequence of the steps 1 to 3 can be reversed.

The use of enzymes occluded in vesicles having an immunological sensitisation, in conjunction with immunosorbents capable of receiving the specific binding of such sensitised vesicles when their immunological valencies are suitably complementary, brings advantage in flexibility and convenience of assay procedure. For example, it enables a separation of solid-phase and liquid-phase to be carried out (either by washing or continuous flow for example) with the handling convenience offered by solid adsorbents, while on release of enzyme from the occluded form even from vesicles which have been fixed to the solid carrier the released enzyme is measurable in solution in the absence of the solid carrier and can retain its full activity. In known solid phase adsorption processes, any adsorbed enzyme has suffered either chemical coupling or avid binding in a way which generally affects both its activity, (whether by denaturation or the kinetic effect of adsorption or steric hindrance, or by more than one of these causes), and its ease of handling. Furthermore, it is easy to arrange for the occluded aqueous environment of the enzyme within the vesicles to be relatively conducive to its satisfactory storage and preservation, and after such occlusion it remains physically relatively unaffected by subsequent stages of the preparation and assay.

Furthermore, materials and methods of this invention can provide a "double amplification" in the number of detectable product molecules relative to the number of analyte molecules which is greater than either the amplification available with single enzyme molecules chemically coupled to immunological reagents or that given by previous attempts to "amplify" enzyme-linked immonoassay in that both the ratio of the molecules of enzyme associated with a given number of molecules of analyte or its complementary reaction component can be higher, and also the state of preservation of activity in those enzyme molecules can be better, i.e. more active, because of their occlusion. during many of the reaction stages, as mentioned above. This can lead to highly satisfactory sensitivity relative to background in a suitable assay configuration.

The vesicles referred to in this specification are in themselves known, as sac-like structures, e.g. liposomes, which are artificial spherules composed of phospholipids arranged to form a bi-lamellar membrane in which polar groups are arrayed externally and and hydrocarbon chains form a hydrophobic core. Liposomes can be prepared according to the method of Gregoriadis et al, FEBS Letters, *14*, 95 (1971) or according to the method of Batzri et al Biochem. Biophys. Acta, *298*, 1015. Because of the hydrophobic core it is possible to introduce a variety of amphipathic antigens into the membrane, to give an immunological valency to the liposomes. One very convenient such material is bacterial lipopolysaccharide, which can conveniently be that obtained from *E. coli* 0149 strains (well known and commercially available).

Further methods of preparing liposomes are referred to in for example US—A—3,850,578, which concerns free-radical-loaded liposomes used in agglutination-type and complement-mediated immunological reactions.

One method of preparing liposomes useful for example in connection with the present invention is to suspend a membrane-forming lipid mixture, comprising phosphatidylcholine, phosphatidylethanolamine and sterol, into an aqueous medium containing bile salt or equivalent detergent, e.g. deoxycholate, any amphiphilic antigen to be incorporated into the sacs, e.g. lipopolysaccharide 0149, and any enzyme to be occluded in the liposomes. The detergent and non-occluded materials can then be removed by gel-filtration. Particular methods of preparing suitable vesicles are described in detail below. The vesicles, e.g. liposomes, can for example have a volume per particle corresponding to diameters of equivalent spheres (or can be roughly spherical with diameters) of about 0.01—10 micrometers.

The methods described herein can result in the occlusion of for example 3 to 30 microliter, e.g. 10 microliter, of enzyme-containing liquid per micromole of lipids used to form the liposomes.

For use in the invention the vesicles have fixed or coupled thereto an immunologically reactive material of specificity appropriate to the determination of interest. This can be achieved for example by fixation of an antigen into the structure of the vesicle, e.g. lipopolysaccharide 0149 of *E. coli*, or by coupling of a desired immunologically reactive material to the vesicle by covalent bonding using a chemical coupling agent or by avid binding, or both. For example, a material desired to be coupled to the vesicle can be coupled to a component of the vesicle, e.g. the amino groups of phosphatidylethanolamine, by using a coupling reagent that reacts with said component to give an activated product that subsequently couples covalently with the material to be coupled. Examples of such coupling reagents are known in themselves and the coupling does not in itself constitute this invention. Suitable coupling reagents include for example dialdehydes such as glutaraldehyde, and adipimidate (see for example GB—A—1,470,955). It is generally desirable to use coupling agents that give rise to linkage groups between vesicle and coupled material that are as long as possible, to avoid steric hindrance, e.g. equivalent to the length of a carbon chain having at least 5 carbon atoms, e.g. up to 20 carbon atoms. Glutaraldehyde gives rise to a linkage group $=CH(CH_2)_3CH=$, i.e. of 5 carbon atoms, and may also give a proportion of linkage groups having longer, condensed, chains. The material to be coupled in this way can be for example an immunoglobulin, a protein or polypeptide antigen, or a non-protein antigen capable of reacting with the coupling agent, e.g. having a free amino-group.

Other coupling methods which can be used, additionally or alternatively, are described in for example US—A—3,817,837 (cols. 31—34) and GB—A—1,316,990 and 1,485,122—3. Additional use of coupling reagents means that the active groups left after reaction of one coupling agent are exposed to reaction themselves with a further coupling reagent.

An alternative or additional and desirable coupling method consists of binding to an immunologically reactive group which itself is fixed or coupled to a vesicle, e.g. by one of the methods just described above, a compound bearing immunological valency of the complementary specificity, optionally itself chemically or physically coupled to a material having a desired further immunological specificity.

For example, liposomes bearing bacterial lipopolysaccharide can be treated with antibody thereto, so that they bear an immunological valency corresponding to the antigenic type of the immunoglobulin constituting the antibody. Alternatively, the liposomes can be reacted with a bifunctional coupled complex, consisting of such an anti-lipopolysaccharide antibody which is chemically coupled to a further antigen or antibody of interest, e.g. anti-(human alpha-fetoprotein), e.g. by reaction with glutaraldehyde, or ethyl N-(carbomoyl-cyano-methyl)-1-iminoacetate followed by diazo-tisation and further coupling (see GB—A—1,316,990). The resulting liposome preparation then has an immunological valency with the specificity of the anti-(human alpha-fetoprotein) or other material coupled to the anti-lipopolysaccharide.

In another example, a protein fixed or coupled to liposomes can have its complementary binding partner coupled thereto by their avid binding reaction, and the resulting complex covalently linked by treatment with dimethyl suberimidate (see Gersten and March-alonis, J. Immunol. Methods (1978) 24 305 for the use of this coupling agent), or an alternative covalent bridging reagent. For example, protein-A from Staphylococcus aureus can be coupled to vesicles using glutaraldehyde, and bound by its characteristic affinity to the Fc group of an immunoglobulin having a desired antibody specificity. The linkage between protein A and the bound antibody can then be made covalent by reaction with the dimethyl suberimidate, to give a vesicle preparation with the specificity of the antibody.

Generally the result of the coupling will be that an immunologically reactive material of desired specificity is coupled to the vesicles, e.g. liposomes, via a linkage comprising a linkage group which is equivalent in length at least to a carbon chain of 5 atoms, e.g. up to 20 atoms; the linkage may comprise a plurality of such linkage groups and/or one or a plurality of protein molecules. The particular coupling methods chosen aim to provide the desired immunological valency sufficiently free of steric hindrance to react with an immunosorbent of suitably complementary valency, as described below.

By way of example, the methods described herein can be used to give attachment of 0.3—10 micrograms, e.g. 1—5 micrograms, of antibody to liposomes for each micromole of lipids used to form the liposomes. Each liposome may have a number of effective binding sites for example in the range 10—100 per liposome.

Alternative vesicle preparations to those noted above can incorporate (instead of bacterial lipopolysaccharide) other amphiphilic antigens, such as erythrocyte membrane antigens and myelin antigens.

In most cases, the result and desired effect is to produce according to the invention a composition of lipid vesicles having an aqueous preparation of an enzyme releasably occluded therein, said vesicles having fixed to their surfaces via a spacer group or spacer molecule a homologue or specific binding partner of an analyte to be detected or determined. (A homologue is understood here to mean a material with specific affinity for a similar binding partner to that of the analyte.) The spacer molecule or group can be for example the residue of a dialdehyde group of at least 5 carbon atoms (or the residue of a coupling reagent of at least equivalent molecule size or length thereto), the residue of dimethyl adipimidate, protein A of S. aureus, or antibody directed against amphipathic lipid such as lipopolysaccharide included in the vesicles. (Together with the solid support materials having surfaces to which are fixed one of the components of the specific binding reaction between two reaction components one of which corresponds to the analyte, and with the lytic agent and the substrate, such compositions form the test kits described herein.)

The methods given above can be used to produce vesicles loaded with enzyme and sensitised by fixation of an immunologically reactive material via a spacer molecule or linkage group sufficiently large to leave the reactive material adequately unhindered sterically for binding to sensitised immunoadsorbent.

The enzyme which is chosen for its occlusion in the vesicles can be any enzyme of choice. The most desirable are those available in quantity with high activity and stability and which posses safe convenient substrates leading to easily measurable products. Hydro-lases, oxidases and reductases provide suitable examples. The most preferred convenient enzyme is alkaline phosphatase for which p-nitrophenyl phosphate is a convenient substrate leading to a convenient easily measurable yellow product, p-nitrophenol. Another convenient enzyme is horseradish peroxidase, usable with substrates hydrogen peroxide and chromogen (see, e.g., GB—A—1,348,935/8).

The nature of the immunoadsorbent is an important feature of the invention, in combination with the remaining material characteristics described herein.

The solid support must be in a form which when immunologically sensitised can accept the binding of immunologically sensitised vesicles having suitable valency.

Preferably the solid support has a solid (i.e. non-porous) surface, e.g. that of a solid polymer such as polystyrene or nylon, to which immunologically reactive material can be coupled. Use can also be made of inorganic solids to which such material can be coupled.

For example, antigen or antibody can be coupled to nylon using adipimidate reagent after partial hydrolysis of nylon to yield amino groups, e.g. as the use of this coupling agent is described in GB—A—1,470,955. Alternatively, antibody can be adsorbed to poly-styrene. This is however somewhat less preferable as it makes certain additional precautions desirable and even in their presence they may be a degree of steric hindrance resulting in lower effective binding capacity of the immunoadsorbent for the sensitised vesicles. A suitable preparation and precautions are for example exposure of the polystyrene surfaces to the antibody (e.g. about 5 $\mu$g/ml) and incubation, e.g. overnight at 37°C, followed by conducting any further reactions with materials which do not include detergent (until it or another lytic agent is finally required to lyse any adherent vesicles) and conducting any further reactions in the presence of ovalbumin (e.g. 1% w/v), or other inert protein to neutralise non-specific adsorption.

Another method of coupling materials to polystyrene is to nitrate the polystyrene to a convenient degree, reduce the nitro groups to amino groups and react the aminopolystyrene with a coupling reagent, e.g. glutaraldehyde, followed by the material to be coupled. A further example of a suitable solid support material is cellulose acetate. Generally, the coupling methods discussed above in connection with vesicle preparations are also applicable to the preparation of immunoadsorbents with any modifications which may be necessitated by the nature of the solid material used as support. Such modifications are in themselves known techniques and form no part of this invention. Applicable methods relating to nylon are described for example by Inman et al., Biochem. J. (1972) *129* 255—262, and Morris et al, Biochem J. (1975) *147*, 593—603. Protein A of *Staphylococcus aureus* is a very suitable coupling material for coupling immunoglobulins both to vesicles and to solid supports using the methods described above. A method for its preparation is described for example in US—A—3,850,798 and its properties are reviewed by J. Langone, in J. Immunol. Methods (1978) *24*, 269—285. One reason for its particular desirability is that it can bind specifically to the Fc parts of immunoglobulin molecules leaving their specific combining sites for their normal binding partners (the Fab parts) unhindered. This orientation can help the capture efficiency of the antibodies used in the assay and test materials.

The form of the solid surfaces carrying the immunoadsorbent reactive groups is generally immaterial in the practice of the invention: convenient forms of solid support are for example tubes, wells, granules, grains, flecks, plates and slides of the material forming the solid support surface.

Where a tube carries a binding reagent or its specific binding partner, it can be made to fit automated continuous-flow equipment so that certain embodiments of the tests and assays can be integrated into automatic analyser systems with exchangeable tube-bound binding reagents.

The lytic agents used according to this invention can be any suitable means for releasing enzyme from its occluded state in the vesicles. Suitable lytic agents include for example detergents, such as Triton X—100, but may consist of hypotonic solutions when the tonicity of the occluded phase is sufficiently high to lead to lysis of the vesicles in more dilute solutions.

The final essential component of the test materials is a substrate for reacting with the occluded enzyme after its release from the vesicles. Any convenient substrate giving a conveniently measurable product is suitable. Examples include p-nitrophenyl phosphate for use with alkaline phosphatase, giving a yellow product p-nitrophenol; 4-methyl-umbelliferyl phosphate for use with alkaline phosphatase giving a highly fluorescent product 4-methyl-umbelliferone; and hydrogen peroxide and any of several chromogens for use with horseradish peroxidase, leading to easily measurable coloured products. Many other examples have been described in connection with enzyme-linked immunoassay systems and are applicable here too.

A number of different assay and test configurations can be employed with materials and methods according to this invention, in addition to those already mentioned.

For example, materials can be provided for a competitive binding assay or test in which enzyme-loaded vesicles bearing a specific binding valency homologous to that of the analyte are provided together with immunoadsorbent having a limiting quantity of the complementary specific binding valency. The assay can then be carried out by mixing the analyte and assay or test materials, separating bound and free vesicles, lysing one of the separated vesicle portions and allowing the liberated enzyme to act on its substrate, yielding a measurable product. Other competitive binding configurations are illustrated for example by the enzyme-coupled assay schemes of US—A—3,654,090, 3,839,153 and 3,850,752. In modifications of these configurations the reactants can be reacted sequentially instead of competing simultaneously.

A preferred assay or test configuration involves test materials in which the enzyme-loaded vesicles and the immunoadsorbent can both bind to the analyte but not to each other. This gives a "sandwich" format: it is of advantage where the .specificity of the vesicles and of the immunoadsorbent can distinguish different determinants on the analyte, e.g. where the analyte is an antibody, one of the specificites can be that of its corresponding antigen, while the other can be that of a corresponding anti-immunoglobulin. The "sandwich" configuration is generally applicable only to antibodies and their binding partners which themselves are of high molecular weight so that they are bivalent, i.e. have two or more specific binding sites. The other configurations are used when low molecular weight binding partners are the analytes, e.g. digoxin and haptens, which generally are monovalent.

The present invention is applicable to the detection and determination of a very large variety of materials that take part in avid specific binding reactions: wherever immunological reagents or reaction components are referred to herein, analogous non-immunological reagents or reaction components are also understood as included. Examples are alpha-fetoprotein, carcinoembryonic antigen, thyroxin binding globulin, pregnancy-associated alpha(2)glycoprotein, antibodies to *Toxoplasma* organisms, hepatitis antigen, rubella antigens, allergens, immunoglobulins E (reagins), *Treponema* antigens, cardiolipin, haemoglobins, immunoglobulins M, digoxin, insulin, human chorionic gonadotrophin, streptococcal antigens, bacterial lipopolysaccharides, and antibodies corresponding to those of the above-mentioned materials that are antigens. Further materials which can be detected and determined by the use of assay materials as described herein and having corresponding specificity are listed for example in US—A—3,996,345.

The detailed preparative and assay procedures and conditions used in conjunction with this invention are in themselves generally those associated with the handling of very small quantities of rather labile biological materials and are in themselves well known. In general, assays are carried out in buffered aqueous media, e.g. pH 5.5 to 10, often 6.5 to 9, the buffers being inert and for present purposes free of detergent at least until the stage of the assay when the lytic agent is called for. Citrate and phosphate are suitable buffer examples. Generally present can be non-detergent inhibitors of non-specific binding phenomena, e.g. inert proteins such as purified ovalbumin from hen's egg, e.g. at 0.1—1% w/v. The buffer solutions can be salinated, e.g. with NaCl to be isotonic with about 0.9% NaCl.

The concentrations of analytes which can be of interest in the assays can be for example in the wide range of about micromolar to fractions of picomolar, e.g. $10^{-6}M$ to $10^{-13}M$ although with certain assays concentrations outside these ranges may be of interest and detectable.

To form the kits of assay materials the quantities of assay materials are chosen to set their quantities in relation to the analyte concentrations of interest. For example a vesicle preparation or immunosorbent which is complementary (and can bind) to the analyte can be present from about 0.1 times the minimum concentration to be detected up to about 100 times the maximum to be detected, based on equivalence of binding capacity; usually up to about 10 times the maximum to be detected.

Another component of the reaction can be present either in excess or in comparable quantity, depending on the chosen assay configuration. Generally the components are present in amounts in which the quantity of sensitised vesicles reacting with the immunoadsorbent is dependent on the quantity of analyte present in the concentration range of interest.

The assays and tests can be carried out so that the binding reactions reach equilibrium, or alternatively so that the amount of product depends on the rate of a critical reaction carried on for a standard time. The reaction times for each assay step where more than one can range from 0.1 minute to about 8 hours, preferably about 1—30 minutes, e.g. 5 to 15 minutes. The required reaction times where a rate-dependent assay is used can be adjusted within limits by choice of reagents concentration: standardised reaction times of 15 seconds to 30 minutes can be used, as standard temperatures, e.g. 15°C—40°C. The order of reagent additions will usually be dictated by the assay configuration chosen.

In carrying out the assays the tests and preparing the test materials, it is desirable to avoid non-specific adsorption of materials important in the assay both to vesicles and to immunoadsorbents, and for this purpose assays can generally be carried out in the presence of a non-detergent inhibitor of non-specific adsorption, e.g. an inert protein (e.g. ovalbumin where this particular material plays no part in the assay in question), and in the absence of detergent until this or another lytic agent is called for to release enzyme from the vesicles at the appropriate time.

It will normally be desirable or necessary, in preparing and using assay and test materials as described herein, to carryout a number of measurements and calibrations of the materials so prepared, in accordance with principles and methods that are well known in themselves. For example it will often be desirable to quantitate the specific binding capacities of the immunoadsorbents and vesicles sensitised by coupling or fixation of immunologically active materials. This can be done e.g. by measuring specific uptake of standardised radiolabelled specific binding partners, or standardised bindable preparations labelled and dosed in other ways.

Calibration of a given assay configuration can be carried out by for example performing parallel tests with serial dilutions plus saturation and null controls in a manner well known in itself. Generally the quantity of product from enzyme liberated from vesicles measured at the end of a given test will show either a direct or inverse relation to the quantity of analyte over a region of dependence on analyte concentration, usually accompanied by a background value. Low background levels can normally be arranged in assay configurations using bivalent analytes, such as large molecular weight antigens and antibodies, in a "sandwich" format in which immunoadsorbent and sensitised vesicles each bind specifically to

**0 014 530**

the analyte but not to each other. Larger background levels are naturally encountered where there is an inverse relation between analyte concentration and final measured enzyme product.

It can be seen in view of the foregoing description and appended examples that the invention also provides a test complex for the detection or determination of an analyte selected from proteinaceous binding reagents and their specific binding partners, said complex comprising said analyte in combination with (a) a reaction component selected from specific binding partners and homologues of the analyte in coupled form to a surface of a solid support material, and (b) a reaction component selected from specific binding partners and homologues of said analyte coupled to the surface of lipid vesicles having releasably occluded therein an aqueous preparation of an enzyme. (In some embodiments the complex will be a "sandwich" type complex with sensitised vesicles bound to the immunoadsorbent via the analyte, and in others the immunoadsorbent will have some of its binding sites occupied by analyte and some occupied by sensitised vesicles of complementary binding specificity.) Also, throughout the specification, where embodiments are given with reference to antigens and antibodies, it is understood that the invention also embraces the complementary alternative embodiments in which the place of antigen is taken by antibody and vice versa.

The following description gives preparative details for some examples of assay materials used according to this invention. It will be apparent that where the preparation refers to antibodies or other specific binding agents, binding agents of alternative specificity can be substituted, to give the manufacture of analogous test materials for the assay of alternative analytes. The preparative details given are only examples of those that enable assays to be carried out in accordance with this invention.

Preparation 1

The following procedure can be used to produce a suspension in aqueous medium of liposomes having entrapped enzyme (in this instance it is alkaline phosphatase) in a separate aqueous phase occluded therein, and incorporating antigenic lipopolysaccharide from *E. coli* 0149 in the liposome structure.

(a) *E. coli* 0149 K91 (L16) "Abbotstown" strain (commercially available) or an equivalent strain of the well-known *E. coli* 0149 is grown on 100 ml of BAB culture medium (4%)+0.1% glucose (w/v) in Roux flasks for 48 hours at 37°C. The resultant bacterial lawn is scraped off the surface in about 2 ml saline per flask and the flasks washed in saline before autoclaving at 121°C and 15 psi ($10^5$ Pa). The autoclaved preparations are centrifuged and a clear supernatant rich in lipopolysaccharide is separated, and concentrated to one-tenth volume by dialysis against polyethylene glycol (20M grade). The pellet from the centrifugation is extracted into an equal volume of phenol:water (90:10 v/v) and is dialysed successively against water (to remove phenol) and polyethylene glycol (to concentrate). The two lipopolysaccharide concentrates are pooled, heated at 56°C in 0.5M NaOH for 1 hour, returned to pH 7 with HCl and dialysed 18 hours against water, before freeze-drying in the presence of 0.1% glucose as cryopreservative. For incorporation in liposomes this lyophilisate is taken up at the rate of 5 mg/ml in citrate or other suitable buffer and sonically disrupted to give a slightly opalescent solution. Equivalent preparations of lipopolysaccharide can be used if desired.

(b) Lipids and aqueous material containing lipopolysaccharide (e.g. from the preparation described above) together with the enzyme to be occluded (alkaline phosphatase, e.g. Sigma Type VII (Trade Mark), about 1:1 w/w in relation to the lipopolysaccharide in standardised activity) are used in the ratio 1 m mole total lipid to 50 ml aqueous material. The lipids used are egg yolk lecithin, cholesterol and L-alpha-phosphatidylethanolamine (molar ratio 7:2:1) dissolved in chloroform at respective concentrations 11.2 mg/ml, 1.6 mg/ml, 0.15 mg/ml. The mixed chloroform solution is evaporated under reduced pressure at 50°C to form a film of solid in the containing vessel, which is then suspended in a volume of 0.1M bicarbonate buffer pH 7.2 equal to that of the chloroform removed, also containing 0.25 mg/ml alkaline phosphatase, 0.24 mg/ml lipopolysaccharide 0149 and 4.2 mg/ml sodium deoxycholate. The milky suspension first obtained is ultrasonically disrupted at 0—4°C until it becomes translucent. At this time the suspension contains mixed micelles of lipid and deoxycholate. To produce liposomes the deoxycholate is removed by gel-filtration on cross-linked agarose (Sepharose 4B (Trade Mark)): the liposomes appear in the eluate close to the void volume.

Inaccessibility to substrate of the occluded enzyme in the product liposomes can be verified by testing aliquots of the liposome preparation (0.1 ml) with 1 ml of pH 9.8 carbonate buffer containing chromogenic substrate (p-nitrophenyl phosphate) 1 mg/ml, in respective tests in the absence and in the presence of a lytic agent and liberating agent for the occluded phase, detergent Triton X—100 (0.1% w/v). The eluate fractions showing highest activity in the presence of detergent are pooled. In the absence of lytic agent only low background levels of enzyme activity are seen.

This or equivalent preparations of liposomes having enzyme occluded therein and bearing antigen on their surfaces (lipopolysaccharide) can be used for assays according to the invention.

The liposome preparation described here is found on electron-microscopic examination under negative staining to comprise particles of average size about 1500 angstroms, composed of one or a few bilayers with large internal spaces.

Preparation 2

Liposomes having material of another immunological specificity than that of lipopolysaccharide of E. coli (i.e. of any desired specificity), bound to their surfaces can be made by the following procedure.

Rabbit or sheep antibody specific for E. coli 0149 lipopolysaccharide is obtained as the IgG fraction of pooled immune rabbit or sheep sera showing high-titre antibody activity against the lipopolysaccharide after immunisation in the normal way.

Sheep antibody specific for human alpha-fetoprotein is obtained in conventional manner (also commercially available, from Seward Laboratory, UAC House, Blackfriars, Road, London SE1, England, under code BN516 (nephelometric grade)).

The anti-(E. coli 0149) is coupled with the anti-(human alpha-fetoprotein) in the following way. Anti-(E. coli 0149) globulin fraction (6 mg/ml) is reacted for several hours at room temperature with 20 mg/ml ethyl N-(carbamyl-cyanomethyl) 1-iminoacetate and dialysed against saline buffer pH 7.2. The resulting antibody solution (derivatised with a substituted 2-amino-1-imidazolyl group) is diazotised at 4°C by the successive addition with mixing of 1.5 volumes of 0.4% aqueous $NaNO_2$, 1.5 volumes of N aqueous HCl and 1.5 volumes of 2% aqueous ammonium sulphamate. After 20 seconds subsequent reaction at 4°C the diazonium product is added dropwise to an excess of sheep anti-(human alpha-fetoprotein) in pH 4.7 buffered saline (10 mg/ml) at 4°C, controlling the (falling) pH to its original value with N aqueous NaOH, and after 10 minutes raising the pH to 7.2. After several hours storage at 4°C and centrifugation, the supernatant containing coupled protein product is taken and stored at −20°C till further use.

One volume of the coupled protein product (about 5 mg/ml), and one volume of the liposome preparation of Preparation 1, are mixed at room temperature 30 minutes, and treated by gel-filtration over cross-linked agarose (Sepharose 4B). Close to the void volume there are eluted fractions containing the product, liposomes as of Preparation 1 having bound thereto coupled complexes of anti-(E. coli 0149)-anti-(human alphafetoprotein). (Uncoupled proteins are separated by the gel filtration emerge in later eluate.) These are suitable for the assay of human alpha-fetoprotein. Instead of the anti-(human alpha-fetoprotein) there can be used in the preparation other antibodies or antigens, such as for example sheep anti-(human IgM/Fc) or sheep anti-(human pregnancy-associated alpha (2) glycoprotein) in which case the resulting product is useful in assays with the corresponding specificity, or the antibody can be substituted with antigen such as, for example, purified human alpha-fetoprotein itself, in which case the product is suitable for use in alternative assay configurations as described above, corresponding to this specificity.

Preparation 3

An alternative procedure for preparing liposomes having material of any desired immunological specificity bound to their surfaces is as follows.

One volume of the liposome suspension obtained by Preparation 1 is reacted with an equal volume of 0.25% w/v glutaraldehyde in 0.1M bicarbonate buffer pH 7.2 for 10 minutes at room temperature and dialysed against 0.1M bicarbonate pH 7.2 for several hours to remove unreacted glutaraldehyde completely. At this point the liposomes bear free aldehyde groups on their surfaces, at the distal end of substituents derived by condensation of one aldehyde group of glutaraldehyde with the ethanolamine amino group of each reacted phosphatidylethanolamine molecule of the liposomes. The activated liposomes are incubated at room temperature (24 hours) with 0.1 mg/ml of the protein of desired immunological specificity to be coupled thereto, e.g. sheep anti-(human alpha-fetoprotein), human alpha-fetoprotein itself, or sheep anti-(human IgE) or other desired antigen or antibody. Finally, excess activation of the liposomes is removed by dialysis of the product against bicarbonate buffer pH 7.2 containing 0.1M ethanolamine for several hours, and the product liposomes bearing coupled reagent of the desired specificity are separated by gel-filtration on cross-linked agarose (Sepharose 4B), eluting the product close to the void volume (identified by assaying aliquots with phosphatase-substrate and detergent). This product contains immunologically active reagent covalently linked to phosphatidylethanolamine molecules of the liposome.

Preparation 4

An alternative procedure for preparing liposomes having immunoglobulin G bound to their surfaces is as follows.

The protein-A from Staphylococcus aureus (which has a binding avidity for immunoglobulin G) is obtained in known manner and coupled to liposomes loaded with alkaline phosphatase in the manner described in Preparations 1 and 3 above. The protein-A is used as the protein to be coupled to the aldehyde-activated liposomes. The so-treated liposomes (in pH 8.5 buffer, 0.1M bicarbonate) are mixed

with an excess (on a protein:coupled protein molar basis, e.g. 0.1 mg protein per ml of liposome preparation) with immunoglobulin G and incubated for several hours up to about a day at room temperature. A tenfold weight excess (based on the immunoglobulin) of dimethyl suberimidate is gradually added to the mixture and if necessary the pH is readjusted to pH 8.5. After 30 minutes at room temperature the mixture is dialysed overnight against carbonate buffer containing 0.1M ethanolamine and separated from low molecular weight reagents and unlinked protein by elution from Sepharose 4B as described in Preparation 1 to 3.

The product has immunoglobulin G of the chosen specificity covalently bound to the liposome by a protein-A bridging group linked covalently both to the immunoglobulin and to the phosphatidylethanolamine molecule of the liposome by glutaraldehyde-derived and suberimidate-derived links respectively, and is useful for assay according to this invention for an analyte corresponding to the specificity of the immunoglobulin used.

## Preparation 5

An alternative method of preparing liposomes bearing lipopolysaccharide (applicable for use also in Preparations 2 to 4 above) is as follows:

Dipalmitoylphosphatidylcholine (83 micromole, 65 mg) is dissolved in each 7.5 ml of isopropyl ether and each 2 ml methanol at 45°C. For each 9.5 ml combined solvent, 2.5 ml of phosphate buffered solution containing 0.5 mg lipopolysaccharide e.g. prepared as described above and 2.5 mg alkaline phosphatase was added. The mixture is thoroughly disrupted ultrasonically (about 15 minutes) at 45°C. During rotary evaporation at 45°C the material forms a semi-solid gel, at which point 4 ml (for each original 20 ml of feed to the evaporator) phosphate buffered saline is added and the evaporation continued 60 minutes more. Finally the residual (5 ml) suspension (for each 4 ml saline added) is subjected to gel filtration (Sepharose 4B) and liposomes containing occluded enzyme detected and harvested from the void volume eluate as described before.

## Preparation 6

Antibodies or antigens can be formed into immunosorbents by linkage to nylon surfaces via glutaraldehyde to give products which can accept the specific binding of suitably activated liposomes, by the following method.

Nylon cups 7 mm by 9 mm diameter, e.g. shaped as standard microtiter trays, or as individual cups, are filled with 3.5M HCl and incubated at 50°C for 1 hour to expose free amino groups by hydrolysis. After thorough washing in phosphate buffered saline (pH 7.2) the cups are filled with glutaraldehyde solution in distilled water (12.5% v/v), chilled to 4° to 6°C. After 30 minutes at 4° to 7°C and thorough washing in phosphate buffered saline, an antibody or antigen to be coupled and itself having free amino groups is added to the cups as a solution at 0.02 to 3 mg/ml concentration and incubated there 16 to 18 hours at 37°C. The buffer for this stage (e.g. pH 7.2 phosphate saline) must be free of any amino compounds capable of competing with the material to be coupled. After the coupling reaction the immunosorbent is prepared for use by washing in plain buffer, and blocking of any residual activated coupling groups by exposure to ethanolamine (1.5M) as the chloride (pH 8.5 with HCl) for 1 hour at 37°C, followed by further washing in plain buffer.

The following Examples further illustrate the invention in conjunction with the description given hereinabove.

## Example 1

(1) Materials according to an example of this invention, suitable for the immunoassay of human alpha-fetoprotein, can be prepared as follows.

A preparation of liposomes having alkaline phosphatase occluded therein, and bearing immunological valency with the specificity anti-(human alpha-fetoprotein) coupled to their surface, is prepared according to Preparations 1, 3 and 4 above (using anti-(human alpha-fetoprotein) (obtainable from Seward Laboratory) as the immunoglobulin G used in Preparation 4). The preparation is kept free of detergent and made 1% w/v with purified ovalbumin. An immunoadsorbent is prepared according to Preparation 6, again using anti-(human alpha-fetoprotein) as the antibody to be coupled. The remaining materials required for the assay are a lytic agent, in this case 0.1% w/v Triton X—100 detergent, and phosphate substrate obtainable commercially as water-soluble pellets, which are dissolved in water before use. Assay is carried out by filling each of a set of similarly-prepared immunoadsorbent cups with dilutions of analyte samples suspected of containing alpha-fetoprotein, dilutions of standard solutions of alpha-fetoprotein (including saturation and null controls), all free of detergent and made 1% w/v in ovalbumin (purified). After standardised 30 minute exposures of cups to samples at 37°C, the analytes are replaced by aliquots of the liposome preparations as described above, and again incubated 30 minutes at 37°C. The cups are then thoroughly washed in pH 7.2 buffer containing 1% w/v purified ovalbumin and then exposed to detergent solution in pH 9.8 carbonate buffer (0.1M). After 2 minutes the detergent solution is transferred to a spectrophotometer cuvette and substrate solution added. The rate of measurable coloured product formation (p-nitrophenol) is taken as the assay result from each cup.

Calibration of the assay is obtained from the series of standard dilutions.

(2) The assay for alpha-fetoprotein given above can be adapted for use as an assay for human chorionic gonadotrophin as follows.

Assay materials are provided as a liposome preparation made as described above in Preparations 1, 3 and 4 above, using anti-(human chorionic gonadotrophin) as the immunoglobulin G in Preparation 4. The preparation was kept free of detergent and made 1% with purified ovalbumin. An immunoadsorbent is prepared according to Preparation 6, using human chorionic gonadotrophin as the antigen to be coupled to the solid support. The remaining assay materials used are as in Part (1) above. However, in this case a limited standardised quantity of the vesicle preparation is used after quantitation of its binding capacity for human chorionic gonadotrophin by standard method. The limited quantity chosen is in excess of the highest concentration (at lowest dilution) of interest in the assay.

The materials are used for assay by exposing the standardised aliquots of vesicles bearing anti-(human chorionic gonadotrophin) to dilutions of the analyte possibly containing the hormone of interest, to dilutions of standard solutions of it, and to saturation and null controls. After 2 hours at 37°C the reaction mixtures are transferred to the immunoadsorbent cups and incubated for a further 2 hours at 37°C. Then the liquid phase is removed from each cup. Either the removed liquid in this case or the remaining well surface can be treated with detergent to release enzyme from the liposomes, and treated to establish corresponding calibration curves and assay results in the usual way. Where the removed liquid is used, the coloured product quantity is directly related to the amount of analyte present, and where the enzyme released from the immunoadsorbed liposomes is used, the quantity of coloured product is inversely related to the amount of analyte present.

Example 2

This Example illustrates materials for the immunoadsorbent immunoassay using enzyme-loaded liposomes in the most simple form.

In summary, liposomes filled with an occluded aqueous preparation of alkaline phosphatase and bearing on their surface alkali-treated lipopolysaccharide extracted from *E. coli*, serotype 0149, were suspended in a solution of ovalbumin in phosphate-buffered saline (PBS) and placed in 300 µl polystyrene wells. The inside walls of the wells had been coated with rabbit-derived antibodies to *E. coli* 0149 organisms. After 90 minutes incubation the wells had been thoroughly rinsed with ovalbumin (1%) in PBS and were then filled with a buffered solution of substrate and detergent. After a further 90 minutes incubation the enzyme/substrate reaction was stopped and the optical densities of the solutions were measured. The system is used for assay by introducing analyte and optionally substituting alternative immunological specificites as described in (1)—(5) below.

The lipopolysaccharide (LPS) was prepared as follows:

Batches of *E. coli*, serotype 0149, K.91 were cultured in 100 ml lots of blood agar No. 2 (Oxoid) containing 0.2% glucose for 48 hours at 37°C. The cells were removed from the surface by the addition of 2 ml saline (0.85%) and sweeping of the surface with a glass rod. The cells were recovered by centrifugation, washed in saline and then suspended in 0.5% formalin for 16 hours. The formaldehyde was removed by dialysis against distilled water.

Lipopolysaccharide was extracted from these killed bacteria by treatment with 90% phenol in water. This was carried out by suspending the killed bacteria in 50 ml of water and mixing this with 50 ml of 90% phenol in water. After being heated at 68°C for 5—10 minutes the mixture was cooled and the top water layer was separated and recovered. This lay was rather thick and had retained much insoluble cell debris; it was necessary to filter it through Whatman No. 1 filter paper before it could be freed of dissolved phenol by dialysis.

The LPS extract was then treated with alkali, to increase its critical micelle concentration (CMC), and so increase its ability to insert into liposome membranes. The suspension was mixed with an equal volume of N sodium hydroxide solution and heated at 56°C for 1 hour. Finally, the extract was neutralised and then dialysed against 0.1% glucose in distilled water in preparation for freeze-drying.

The antibodies were obtained as follows:

The antibodies used were in the form of IgG fractions from the sera of rabbits immunised with *E. coli* 0149. The IgG fractions were prepared from whole sera by (1) double precipitation with sodium sulphate (to 16%) and (2) subsequent ion exchange chromatography on Whatman DE.52 cellulose.

The immunosorbent was produced as follows:

Immunosorbent surfaces were prepared by attaching rabbit anti-0149 antibodies onto the inside walls of Dynatech polystyrene "Removawells". This was achieved by simply filling the wells with antibody solution in pH 9.8 carbonate buffer at a total protein concentration of 5 µg per ml, and incubating them in a sealed container at 37°C overnight.

The enzyme-filled, antigen-sensitised liposomes were prepared as follows:

Liposomes were prepared from a lipid mixture containing lecithin (16.42 mg), chlesterol (5.80 mg) and dicetyl phosphate (1.20 mg) giving a molar ratio of 2:1.5:0.22. The molar quantity of lecithin used for this preparation of liposomes was 20µ moles. Alkali-treated LPS (120 µg) and alkaline phosphatase (1,500 µg) were included in the aqueous suspending fluid to provide sensitising antigen and marker enzyme.

Firstly, the lipid components were dissolved in 25 ml of chloroform and were deposited on the inside surface of a 100 ml round bottom flask, by rotary evaporation under reduced pressure at 40°C. Last traces of solvent were removed by placing the flask under high vacuum for 1 hour.

The dried lipid film was suspended in a 2 ml solution of LPS (120 $\mu$g) and alkaline phosphatase (Sigma Type VII, 1,500 $\mu$g) in PBS. To facilitate dispersion of the lipids, the mixture was agitated by means of "Whirlimix" Vortex Mixer and the milky-white suspension was left to stand overnight at 4°C.

In the preparation of the aqueous suspending medium it was necessary to dissolve LPS in a standard, stock solution by taking up 5 mg of the freeze-dried material in 1 ml of distilled water and subjecting this to sonication until it was only slightly opalescent. Of this stock solution, 24 $\mu$ ml were added to the aqueous suspending medium to give the required quantity of 120 $\mu$g.

Free (unentrapped) enzyme was removed from the suspension by chromatography on Sepharose 4B. The liposomes eluted from the column in the void volume but free enzyme was retained and came out in latter fractions. To confirm the presence of trapped enzyme in these liposomes a 5 $\mu$l sample was mixed with substrate solution (nitro phenyl phosphate, 1 mg/ml in carbonate buffer, pH 9.8) and the amount of product formation after one hour was measured in terms of the optical density (OD) at 400 nm. A second sample was set up in an identical system but in the presence of 1% Triton X—100. In the absence of Triton X—100 there was only a very slight development of colour, but in its presence there was an intense colour development after only a few minutes' incubation, so indicating the entrapment of a large amount of enzyme (see Table 1).

Table 1

Free and entrapped enzyme in the liposome suspension after elution from sepharose 4B

| Volume of liposome suspension | OD after 60 minutes with: | |
| --- | --- | --- |
| | Substrate+triton | Substrate alone |
| 5 $\mu$l | 1.887 | 0.044 |
| 0.5 $\mu$l | 0.214 | 0.007 |

After separation on Sepharose 4B the liposomes were suspended in 15 ml of PBS.

Assay procedure for specific binding

Dynatech "Removawells", one set having been pre-treated with antibodies to *E. coli* 0149, and the other having been left untreated as a negative control were washed 3 times with 1% ovalbumin in PBS. An appropriately diluted sample of the liposome suspension was placed in each well and they were incubated at 37°C for 90 minutes. The sample volume added in each case was 50 $\mu$l.

After the first incubation, the wells were emptied and then washed twice in 1% ovalbumin in PBS. The substrate (p-nitrophenyl phosphate, 1 mg/ml) was dissolved in carbonate buffer pH 9.8 containing 1% Triton X—100 and this was placed into each well at the rate of 250 $\mu$l per well. After another 90 minutes incubation the enzyme reaction was stopped by the addition of 50 $\mu$l of 2N sodium hydroxide and the optical densities at 400 nm were recorded.

The results obtained from the specific binding assay show the liposomes can be bound by immobilised antibodies, through surface membrane antigens. This binding is specific and is firm enough to withstand a relatively vigorous rinsing process. The specificity is confirmed by the absence of binding, even when a non-specific protein (i.e. ovalbumin) is included in the sample, thus precluding the possibility of non-specific adsorption of liposomes onto a layer of adsorbed protein at the polystyrene/water interface.

Finally, the enzyme activity detected in those wells bearing antibodies to 0149 LPS can only be attributed to the binding of liposomes through their surface LPS. The release of this activity upon incubation with substrate and detergent confirms that such bound liposomes can be made to yield their contents quantitatively, as needed.

It is apparent that the test materials described above (immunosorbent, and liposomes bearing antigenic lipopolysaccharide), are applicable in immunoassay according to the procedures described above, in any of several forms, for example as follows.

(1) Antibody against *E. coli* 0149 or its lipopolysaccharide can be assayed by exposing aliquots of the liposomes bearing the lipopolysaccharide to contact with the antibody under assay before placement in the wells. Any antibody present in the material under assay depresses the extent of adherence of liposomes to the well walls. Detection of the vesicles and estimation of their amount is carried out by detergent lysis, exposure to substrate, and optical measurement, as described above. Control and calibration are performed in the usual way.

(2) *E. coli* 0149 or its native lipopolysaccharide can be assayed by contacting the material under assay with the immunoadsorbent (well walls coated with antibody) before or during exposure of the immunosorbent to the liposomes. Any antigen present in the material under assay depresses the extent of adherence of liposomes to the well walls. Detection and estimation are carried out as before. Control and calibration are performed in the usual way.

(3) Antibody against *E. coli* O149 or its lipopolysaccharide can also be assayed using a modified immunosorbent in the system to give a "sandwich" technique. Instead of using antibody against *E. coli* O149 to coat the well walls, a heterologous anti-immunoglobulin G or (even better) anti-Fc globulin directed against the species from which the material under assay comes, can be used to coat the walls. The material under assay is then exposed first either to the liposomes, or to the well walls, or it is placed in the wells in admixture with the liposomes: then the extent of adherence of liposomes to the well walls is directly related to the amount of anti-*E. coli* O149 present in the test material. Detection and estimation are carried out as before. Control and calibration are performed in the usual way.

(4) It is also apparent that the test materials described above can be applied to the immunoassay of for example the human alpha-fetoprotein mentioned above. In the manner described above, the preparation of liposomes or vesicles bearing the preparation of a bi-functional complex consisting of antibody specific against *E. coli* O149 or against its lipopolysaccharide, coupled as mentioned above using the coupling technique of GB—A—1,316,990, to a heterologous antibody raised against human alpha-fetoprotein, e.g. raised in the sheep. This fixes the bi-functional complex on to the surface of the vesicles, and aliquots of the resulting preparation can be exposed to material under assay possibly containing human alpha-fetoprotein, and to an immunosorbent as described above which is specific for alpha-fetoprotein. The immunosorbent can be made by using an antibody to human alpha-fetoprotein (instead of the anti-*E. coli* O149) to coat the wells in the above-described immunosorbent preparation. Then the extent of adherence of liposomes to the walls of the well is directly related to the amount of human alpha-fetoprotein in the material under assay. Detection and estimation are carried out as described above. Control and calibration are performed in the usual way.

(5) It is apparent that all the immunosorbents described above in this example can alternatively be made in the manner described above by coupling the respective antibody with the surface of a nylon tube activated by acid hydrolysis and reacted with adipimidate reagent (as described in GB—A—1,470,955 mentioned above).

The following expressions used in this description are Trade Marks:—
Triton X—100, Sigma Type VII, Sepharose 4B, Seward Laboratory, Dynatech Removawells.

**Claims**

1. A kit of test materials for carrying out detection or determination of an analyte which corresponds to one component of an avid specific binding reaction between two reaction components comprising a proteinaceous binding reagent and its specific binding partner, said kit comprising a solid adsorbent comprising one of said reaction components, an enzyme-labelled one of said reaction components, and a substrate for reacting with said enzyme to give a detectable product, characterised in that the kit comprises the following further three ingredients:

(a) lipid vesicles having releasably occluded therein an aqueous preparation of the enzyme, said lipid vesicles having fixed to their surfaces one of said reaction components;
(b) a solid support material having a surface to which one of said components is fixed, and able to accept the binding of component (a) when this is of complementary valency; and
(c) a lytic agent for lysing said vesicles and releasing the occluded enzyme therefrom.

2. A kit of test materials according to claim 1, characterised in that one reaction component is fixed to said vesicle surfaces or fixed to the surface of the solid support material through a spacer molecule or group selected from linkage groups having at least the length of a chain of 5 carbon atoms, and protein molecules; being preferably the residue of a dialdehyde group having at least 5 carbon atoms; the residue of dimethyl adipimidate; protein A of *Staphylococcus aureus*; or antibody directed against amphipathic lipid forming part of said vesicle.

3. A kit of test materials according to claim 1 or 2, wherein said reaction components are selected from human alpha fetoprotein, human chorionic gonadotrophin, hepatitis antigen, immunoglobulins, and antibodies specific thereto.

4. A kit of test materials according to any one of claims 1—3, wherein said one reaction components fixed to said vesicles and said one component fixed to said solid support material are free of binding specificity for each other and each has a binding specificity for said analyte.

5. A kit of test materials according to any one of claims 1—4, wherein said one reaction component fixed to said vesicles and said one component fixed to said solid support have complementary binding specificity to each other.

6. A composition comprising a test complex as produced by the use of a test kit according to claim 1, for the detection or determination of an analyte selected from proteinaceous binding reagents and their specific binding partners, said complex comprising said analyte in combination with

(a) a reaction component selected from specific binding partners and homologs of said analyte in coupled form to a surface of a solid support material and
(b) a reaction component selected from specific binding partners and homologs of said analyte coupled to the surface of lipid vesicles having an aqueous preparation of an enzyme releasably occluded therein.

7. A test method comprising the use of a test kit according to claim 1, for the detection or determination of an analyte which corresponds to one component of an avid binding reaction between two reaction components comprising a proteinaceous binding reagent and its specific binding partner, said method comprising reacting said analyte with assay materials including

(a) lipid vesicles having releasably occluded therein an aqueous preparation of an enzyme, said lipid vesicles having fixed to their surfaces one of said reaction components; and
(b) a solid support material having a surface to which one of said components is fixed in amounts in which the quantity of (a) reacting with (b) is dependent on the quantity of analyte present; separating from said reaction a solid or liquid phase containing an amount of said (a) dependent on the quantity of analyte present; treating said separated phase with a lytic agent to lyse said vesicles and release the occluded enzyme therefrom; and contacting said released enzyme with a substrate to provide a measurable product in dependence on the amount of said released enzyme.

8. A test method according to claim 7, characterised in that one reaction component is fixed to said vesicle surfaces or fixed to the surface of the solid support material through a spacer molecule or group selected from linkage groups having at least the length of a chain of 5 carbon atoms, and protein molecules, and preferably the residue of a dialdehyde group having at least 5 carbon atoms; the residue of dimethyl adipimidate; protein A of *Staphylococcus aureus*; and antibody directed against amphipathic lipid forming part of said vesicle.

9. A test method according to claim 7 or 8, characterised in that said one reaction component fixed to said vesicles and said one component fixed to said solid support material are free of binding specificity for each other and each has a binding specificity for said analyte.

10. A test method according to claim 7, 8 or 9, characterised in that said one reaction component fixed to said vesicles and said one component fixed to said solid support have complementary binding specificity to each other.

**Revendications**

1. Une trousse de matériaux d'examen pour effectuer la détection ou la détermination d'un composé à analyser qui correspond à un composant d'une réaction de liaison spécifique par affinité entre deux composants réactionnels comprenant un réactif de liaison protéinique et son partenaire liant spécifique, ladite trousse comprenant un adsorbant solide comprenant un desdits composants réactionnels, un desdits composants réactionnels marqués par une enzyme, et un substrat pour réagir avec ladite enzyme pour fournir un produit détectable, la trousse étant caractérisée en ce qu'elle comprend de plus les trois ingrédients suivants:

(a) des vésicules lipidiques renfermant de façon à pourvoir la libérer une préparation aqueuse de l'enzyme, lesdites vésicules lipidiques ayant un desdits composants réactionnels fixé à leurs surfaces;
(b) une matière support solide ayant une surface à laquelle un desdits composants est fixé et capable d'accepter la liaison du composant (a) lorsque celui-ci est de valence complémentaire; et
(c) un agent lytique pour lyser lesdites vésicules et en libérer l'enzyme contenue.

2. Une trousse de matériaux d'examen selon la revendication 1, caractérisée en ce qu'un des composants réactionnels est fixé auxdites surfaces des vésicules ou fixé à la surface de la matière support solide par une molécule d'espacement ou un groupe choisi parmi les groupes de liaison ayant au moins la longueur d'une chaîne de 5 atomes de carbone, et les molécules protéiniques; qui est de préférence le reste d'un groupe dialdéhyde ayant au moins 5 atomes de carbone; le reste de l'adipidimate de diméthyle; la protéine A et *Staphylococcus aureus*; ou un anticorps dirigé contre un lipide amphipathique faisant partie de la surface de ladite vésicule.

3. Une trousse de matériaux d'examen selon la revendication 1 ou 2, dans laquelle lesdits composants réactionnels sont choisis parmi l'$\alpha$-foetoprotéine humaine, la gonadotrophine chorionique humaine, l'antigène de l'hépatite, les immunoglobulines et les anticorps qui leur sont spécifiques.

4. Une trousse de matériau d'examen selon l'une quelconque des revendications 1 à 3 dans laquelle ledit composant réactionnel fixé auxdites vésicules et ledit composé fixé à ladite matière support solide sont libres de spécificité de liaison mutuelle et ont chacun une spécificité de liaison pour ledit composé à analyser.

5. Une trousse de matériau d'examen selon l'une quelconque des revendications 1 à 4, dans laquelle ledit composant réactionnel fixé auxdites vésicules et ledit composant fixé audit support solide ont mutuellement une spécificité de liaison complémentaire.

6. Une composition comprenant un complexe d'examen produit par l'emploi d'une trousse d'examen selon la revendication 1 pour la détection ou la détermination d'un composé à analyser choisi parmi les réactifs protéiniques liants et leurs partenaires liants spécifiques, ledit complexe comprenant ledit composé à analyser en combinaison avec:

(a) un composant réactionnel choisi parmi les partenaires liants spécifiques et les homologues dudit composé à analyser sous une forme couplée à une surface d'une matière support solide et

(b) un composant réactionnel choisi parmi les partenaires liants spécifiques et les homologues dudit composé à analyser couplé à la surface de vésicules lipidique contenant, de façon à pouvoir la libérer, une préparation aqueuse d'une enzyme.

7. Un procédé d'examen comprenant l'emploi d'une trousse d'examen selon la revendication 1, pour la détection ou la détermination d'un composé à analyser qui correspond à un composant d'une réaction de liaison par affinité entre deux composants réactionnels comprenant un réactif liant protéinique et son partenaire liant spécifique, ledit procédé comprenant la réaction dudit composé à analyser avec des matériaux d'examen comprenant:

(a) des vésicules lipidiques contenant, de façon à pouvoir la libérer une préparation aqueuse d'une enzyme, lesdites vésicules lipidiques ayant un desdits composants réactionnels fixé à leurs surfaces; et

(b) une matière support solide ayant une surface à laquelle est fixé un desdits composants en des quantités telles que la quantité de (a) réagissant avec (b) dépende de la quantité du composé à analyser présent; la séparation à partir de ladite réaction d'une phase solide ou liquide contenant une quantité dudit (a) dépendant de la quantité du composé à analyser présent; le traitement de ladite phase séparée avec un agent lytique pour lyser lesdites vésicules et en libérer l'enzyme contenue; et le contact de ladite enzyme libérée avec un substrat pour fournir un produit mesurable selon la quantité de ladite enzyme libérée.

8. Un procédé d'examen selon la revendication 7, caractérisé en ce qu'un des composants réactionnels est fixé aux surfaces desdites vésicules ou est fixé à la surface de la matière support solide par l'intermédiaire d'une molécule d'espacement ou d'un groupe choisi parmi les groupes de liaison ayant au moins la longueur d'une chaîne de 5 atomes de carbone, et les molécules protéiniques, et de préférence le reste d'un groupe dialdéhyde ayant au moins 5 atomes de carbone; le reste de l'adipimidate de diméthyle; la protéine A de *Staphylococcus aureus*; et un anticorps dirigé contre un lipide amphipathique faisant partie de la surface de ladite vésicule.

9. Un procédé d'examen selon la revendication 7 ou 8, caractérisé en ce que ledit composant réactionnel fixé auxdites vésicules et ledit composant fixé à ladite matière support solide sont dépourvus de spécificité de liaison mutuelle et ont chacun une spécificité de liaison pour ledit composé à analyser.

10. Un procédé d'examen selon l'une des revendications 7, 8 ou 9, caractérisé en ce que ledit composant réactionnel fixé auxdites vésicules et ledit composant fixé audit support solide ont une spécificité mutuelle de liaison complémentaire.

**Patentansprüche**

1. Ausrüstung von Testmaterialien zum Durchführen des Nachweises oder der Bestimmung eines Analyten, der einer Komponente einer aviden spezifischen Bindungsreaktion zwischen zwei Reaktionskomponenten entspricht, die ein proteinhaltiges Bindemittel und dessen spezifischen Bindungspartner umfassen; welche Ausrüstung ein festes Adsorbens, das eine der Reaktionskomponenten darstellt, eine enzymmarkierte Reaktionskomponente und ein Substrat zum Umsetzen mit dem Enzym umfaßt, um ein nachweisbares Produkt zu ergeben, dadurch gekennzeichnet, daß die Ausrüstung die folgenden weiteren drei Bestandteile aufweist:

(a) Lipidvesikeln mit einer darin freisetzbar okkludierten wässerigen Zubereitung des Enzyms, welche Lipidvesikeln an ihren Oberflächen eine der Reaktionskomponenten fixiert aufweisen,

(b) ein festes Trägermaterial mit einer Oberfläche, an welche eine der Komponenten fixiert ist, und die imstande ist, die Bindung der Komponente (a), wenn diese eine komplementäre Valenz aufweist, anzunehmen, und

(c) ein lytisches Mittel zum Lösen der Vesikeln und Freisetzen des okkludierten Enzyms daraus.

2. Ausrüstung von Testmaterialien gemäß Anspruch 1, dadurch gekennzeichnet, daß eine Reaktionskomponente an die Vesikeloberflächen fixiert oder an die Oberfläche des festen Trägermaterials durch ein Abstandsmolekül oder ein Abstandsgruppe, ausgewählt unter Bindungsgruppen mit mindestens der Länge einer Kette mit 5 C-Atomen und Proteinmolekülen, die bzw. der vorzugsweise der Rest einer Dialdehydgruppe mit mindestens 5 C-Atomen, der Rest von Dimethyladipimidat; Protein A von Staphylococcus aureus; oder gegen amphipathisches Lipid, das einen Teil der Oberfläche des Vesikels bildet, gerichteter Antikörper ist, fixiert ist.

3. Ausrüstung von Testmaterialien gemäß Anspruch 1 oder 2, wobei die Reaktionskomponenten unter menschlichem $\alpha$-Fetoprotein, menschlichem Choriongonadotrophin, Hepatitisantigen, Immunoglobulinen und anderen dagegen spezifischen Antikörpern gewählt sind.

4. Ausrüstung von Testmaterialien gemäß einem der Ansprüche 1 bis 3, wobei die eine der Reaktionskomponenten, die an die Vesikeln fixiert ist, und die eine Komponente, die an das feste Trägermaterial fixiert ist, frei von Bindungscharakter in bezug aufeinander sind und jeweils einen Bindungscharakter für den Analyten aufweisen.

5. Ausrüstung von Testmaterialien gemäß einem der Ansprüche 1 bis 4, wobei die eine Reaktionskomponente, die an die Vesikeln fixiert ist, und die eine Komponente, die an den festen Träger fixiert ist, in bezug aufeinander komplementären Bindungscharakter aufweisen.

6. Zusammensetzung, umfassend einen Testkomplex, wie er durch die Verwendung einer Testausrüstung gemäß Anspruch 1 gebildet ist, für den Nachweis und die Bestimmung eines Analyten ausgewählt unter proteinhaltigen Bindungsreagentien und ihren spezifischen Bindungspartnern, welcher Komplex den Analyten· in Kombination mit

(a) einer Reaktionskomponente ausgewählt unter spezifischen Bindungspartnern und Homologen des Analyten in einer an eine Oberfläche eines festen Trägermaterials gekoppelten Form und

(b) einer Reaktionskomponente ausgewählt unter spezifischen Bindungspartnern und Homologen des Analyten gekoppelt an die Oberfläche von Lipidvesikeln mit einer darin freisetzbar okkludierten wässerigen Zubereitung eines Enzyms, umfaßt.

7. Testverfahren, umfassend die Verwendung einer Testausrüstung gemäß Anspruch 1 zum Nachweisen oder Bestimmen eines Analyten, der einer Komponente einer aviden Bindungsreaktioh zwischen zwei Reaktionskomponenten entspricht, die ein proteinhaltiges Bindungsreagens und dessen spezifischen Bindungspartner umfassen, welches Verfahren die Umsetzung des Analyten mit Untersuchungsmaterialien einschließlich

(a) Lipidvesikeln mit einer darin freisetzbar okkludierten wässerigen Zubereitung eines Enzyms, welche Lipidvesikeln an ihren Oberflächen fixiert eine der Reaktionskomponenten aufweisen, und

(b) eines festen Trägermaterials mit einer Oberfläche, an welche eine der genannten Komponenten fixiert ist, in Anteilen, in welchen die Quantität von (a), die mit (b) reagiert, von der Quantität des vorhandenen Analyten abhängig ist; das Abtrennen einer festen oder flüssigen Phase, die einen Anteil an (a) in Abhängigkeit von der Quantität an vorhandenem Analyten enthält, von der Reaktion; das Behandeln der abgetrennten Phase mit einem lytischen Mittel zum Lösen der Vesikeln und Freisetzen des okkludierten Enzyms daraus; und das Inberührungbringen des freigesetzten Enzyms mit einem Substrat, um in Abhängigkeit vom Anteil des freigesetzten Enzyms ein meßbares Produkt zu erhalten, umfaßt.

8. Testverfahren gemäß Anspruch 7, dadurch gekennzeichnet, daß eine Reaktionskomponente an die Vesikeloberflächen oder an die Oberfläche des festen Trägermaterials durch ein Abstandsmolekül oder eine Abstandsgruppe, ausgewählt unter Bindungsgruppen mit mindestens der Länge einer Kette mit 5 C-Atomen und Proteinmolekülen, vorzugsweise den Rest einer Dialdehydgruppe mit mindestens 5 C-Atomen; den Rest von Dimethyladipimidat; Protein A von Staphylococcus aureus; und gegen amphipathisches Lipid, das einen Teil der Oberfläche des Vesikels bildet, gerichteten Antikörper, fixiert ist.

9. Testverfahren gemäß Anspruch 7 oder 8, dadurch gekennzeichnet, daß die eine Reaktionskomponente, die an die Vesikeln fixiert ist, und die eine Komponente, die an das feste Trägermaterial fixiert ist, frei von Bindungscharakter in bezug aufeinander sind und jeweils einen Bindungscharakter für den Analyten aufweisen.

10. Testverfahren gemäß Anspruch 7, 8 oder 9, dadurch gekennzeichnet, daß die eine Reaktionskomponente, die an die Vesikeln fixiert ist, und die eine Komponente, die an den festen Träger fixiert ist, in bezug aufeinander komplementären Bindungscharakter aufweisen.